(19) Europäisches Patentamt
European Patent Office
Office européen des brevets

(11) Veröffentlichungsnummer: **0 323 593 B1**

(12) **EUROPÄISCHE PATENTSCHRIFT**

(45) Veröffentlichungstag der Patentschrift: **16.09.92**

(51) Int. Cl.[5]: **C07D 233/32**, C07D 239/10, B01D 11/02

(21) Anmeldenummer: **88120963.9**

(22) Anmeldetag: **15.12.88**

(54) **Verfahren zur Herstellung von N,N'-dialkylsubstituierten cyclischen Harnstoffderivaten.**

(30) Priorität: **24.12.87 DE 3744120**

(43) Veröffentlichungstag der Anmeldung:
**12.07.89 Patentblatt 89/28**

(45) Bekanntmachung des Hinweises auf die Patenterteilung:
**16.09.92 Patentblatt 92/38**

(84) Benannte Vertragsstaaten:
**DE FR GB**

(56) Entgegenhaltungen:
**EP-A- 0 280 781**
**US-A- 2 497 308**
**US-A- 4 642 351**

**CHEMICAL ABSTRACTS, Band 98, Nr. 15, 11. April 1983, Seite 642, Nr. 126094a, Columbus, Ohio, US; & JP-A-57 175 170**

(73) Patentinhaber: **BASF Aktiengesellschaft**
**Carl-Bosch-Strasse 38**
**W-6700 Ludwigshafen(DE)**

(72) Erfinder: **Schoenleben, Willibald, Dr.**
**Blumenthalstrasse 41**
**W-6900 Heidelberg(DE)**

EP 0 323 593 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von N,N'-dialkylsubstituierten cyclischen Harnstoffderivaten der allgemeinen Formel I

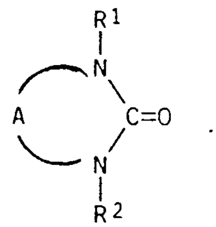

(I),

in der die Reste $R^1$ und $R^2$ Alkylreste mit 1 bis 8 C-Atomen und A eine $CR^3R^4$-$CR^5R^6$- oder eine $CR^3R^4$-$CR^5R^6$-$CR^7R^8$-Gruppe bedeutet, wobei $R^3$ bis $R^8$ für Wasserstoff oder Alkylreste mit 1 bis 17 C-Atomen stehen, durch Umsetzung von Diaminen der allgemeinen Formel II

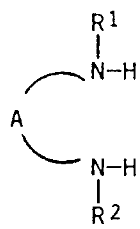

(II),

mit Kohlendioxid in der Gasphase in Gegenwart von oxidischen Katalysatoren.

Diese Verbindungen können auch als cyclische Harnstoffderivate aufgefaßt werden.

Die N,N'-dialkylsubstituierten cyclischen Harnstoffderivate sind als polare, aprotische Lösungsmittel von technischem Interesse. Sie werden zum Lösen von Arzneimitteln, von hochmolekularen Substanzen wie Polyamiden, PVC, Polystyrol, Polyurethan usw. und von anorganischen Verbindungen verwendet und dienen als Medien bei chemischen Reaktionen.

Es sind zwar einige Methoden zur Herstellung der N,N'-dialkylsubstituierten cyclischen Harnstoffderivaten bekannt, diese benötigen aber teure oder schwer handhabbare Ausgangsstoffe oder geben nur mäßige Ausbeuten. Eine Übersicht über die Herstellmethoden findet sich bei Shenoy und Pearce (Am. Dyestuff Rep. 57, 1968, 352). Beispielsweise kann man Diamine vom Typus des N,N'-Dimethylethylendiamins mit Harnstoff, Diethylcarbonat oder Phosgen zum 5-Ring cyclisieren.

Noch günstiger als Harnstoff wäre die Verwendung von Kohlendioxid als billiges, leicht handhabbares Cyclisierungsmittel. Es ist zwar bekannt, Ethylendiamin und $CO_2$ thermisch bei 200-300 °C zu Ethylenharnstoff umzusetzen (z.B. US 2 497 308 sowie Mulvaney und Evans, Ind. Eng. Chem. 40, 1948, 393). Im Falle der N-substituierten Diamine versagt die rein thermische Methode jedoch. Deshalb katalysieren Nomura et al (Ind. Eng. Chem. Res. 1987, 1056-1059) die Reaktion mit Triphenylstibinoxid $Ph_3SbO$ in Gegenwart von Zeolith und erhalten 1-Methyl-2-imidazolidinon, allerdings nur in 85 %iger Ausbeute.

Nach der japanischen Offenlegungsschrift 57/175 170 (Chem. Abstracts 98, 126094a) wird 1,3-Dimethyl-2-imidazolidinon durch Erhitzen von N,N'-Dimethylethylendiamin und $CO_2$ in Gegenwart von Methylamin auf 210 °C im Autoklav hergestellt. Die Ausbeute beträgt allerdings nur 85 % der Theorie.

Es bestand die Aufgabe, ein Verfahren zu finden, nach dem N,N'-dialkylsubstituierte cyclische Harnstoffderivate durch Umsetzung von N,N'-Dialkyldiaminen II mit Kohlendioxid in guten Ausbeuten auf wirtschaftliche Weise erhalten werden können. Weiterhin bestand die Aufgabe 1,3-Dialkyl-2-imidazolidinone zur Verfügung zu stellen, die bei tiefen Temperaturen als polare Lösungsmittel für hochmolekulare Verbindungen verwendet werden können.

Demgemäß wurde gefunden, daß man N,N'-dialkylsubstituierte cyclische Harnstoffderivate der Formel I

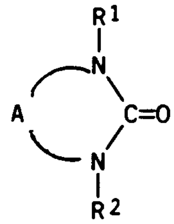

(I),

in der die Reste $R^1$ und $R^2$ Alkylreste mit 1 bis 8 C-Atomen und A eine $CR^3R^4$-$CR^5R^6$- oder eine $CR^3R^4$-$CR^5R^6$-$CR^7R^8$-Gruppe bedeutet, wobei $R^3$ bis $R^8$ für Wasserstoff oder Alkylreste mit 1 bis 17 C-Atomen stehen, durch Umsetzung von Diaminen der allgemeinen Formel II

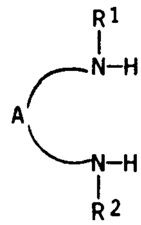

(II),

mit Kohlendioxid in der Gasphase vorteilhaft herstellen lassen, indem man die Umsetzung in Gegenwart von Oxiden von Elementen der dritten oder vierten Hauptgruppe oder zweiten bis sechsten Nebengruppe des Periodischen Systems oder Gemischen hiervon oder in Gegenwart von Aluminium- oder Magnesiumsilikaten durchführt.

Für das erfindungsgemäße Verfahren in Frage kommende Katalysatoren sind z.B. acide, oxidische Heterogenkatalysatoren sowie insbesondere oxidische Verbindungen, die sowohl saure als auch basische Eigenschaften aufweisen wie beispielsweise Aluminiumoxide, insbesondere $\gamma$-$Al_2O_3$, Aluminiumsilikate oder Zinkoxid. Anstelle von Aluminiumsilikaten kommen auch Magnesiumsilikate oder Gemische von Aluminium- und Magnesiumsilikaten in Betracht. Anstelle der oxidischen Verbindungen direkt können auch Verbindungen verwendet werden, die unter den Reaktionsbedingungen in die Oxide überführt werden, z.B. Aluminiumhydroxide oder -oxidhydrate.

Beispielhaft seien folgende oxidische Katalysatoren aufgeführt: Siliciumdioxid in Form von Kieselgel, Kieselgur, Quarz, Aluminiumoxide, Boroxide, Zinkoxid, Titandioxid, Zirkondioxid, Vanadiumoxide, Nioboxide, Chromoxide, Molybdänoxide, Wolframoxide oder Bims oder Gemische dieser Oxide wie $SiO_2 \cdot Al_2O_3$, $B_2O_3 \cdot Al_2O_3$, $Cr_2O_3 \cdot Al_2O_3$, $MoO_3 \cdot Al_2O_3$ oder $ZrO_2 \cdot SiO_2$.

Als Katalysatoren können auch zeolithische Katalysatoren verwendet werden. Zeolithe sind kristalline Aluminosilikate, die eine hochgeordnete Struktur mit einem starren dreidimensionalen Netzwerke von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2 (siehe Ullmanns Encyclopädie d. techn. Chemie, 4. Auflage, Band 24, Seite 575 1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (s. Ullmanns Encyclopäidie d. techn. Chemie, 4. Aufl., Band 24, Seite 575, 1983). So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind z. B. Zeolithe aus der Mordenit-Gruppe oder engporige Zeolithe vom Erionit- bzw. Chabasit-Type oder Zeolithe vom Faujasit-Typ, z.B. Y-, X- oder L-Zeolithe sowie Pentasilzeolithe, die als Grundbaustein einen aus $SiO_4$-Tetraedern aufgebauten Fünfring gemeinsam haben. Sie sind durch ein hohes $SiO_2/Al_2O_3$-Verhältnis von etwa 10 bis 40 000 gekennzeichnet sowie durch Porengrößen, die zwischen denen der Zeolithe vom Typ A und denen

vom Typ X oder Y liegen. Ihre Herstellung ist z.B. in der DE-OS 3 513 725 oder DE-OS 35 06 632 beschrieben.

Die erfindungsgemäß verwendbaren Katalysatoren können durch Behandlung mit Säuren, z.B. Phosphorsäure oder Polyphosphorsäure modifiziert werden. Beispielsweise kann man Phosphorsäure auf $SiO_2$-, $Al_2O_3$- oder Bims-Träger durch Auftränken oder Versprühen aufbringen. Anschließend wird in der Regel getrocknet bzw. calciniert.

Die oxidischen Katalysatoren können wahlweise als Stränge (z.B. 2-4 mm Länge), als Tabletten (z.B. 3-5 mm Durchmesser) oder als Splitt eingesetzt werden. Anstelle der Festbettfahrweise können die Katalysatoren auch als Wirbelkontakte verwendet werden.

Die Umsetzung am Katalysator wird in der Gasphase, in der Regel bei Temperaturen von 200 bis 350 °C, insbesondere 230 bis 300 °C vorgenommen.

Die Reaktion vollzieht sich in 2 Teilschritten, der leicht ablaufenden Bildung einer Carbamidsäure und deren katalytischer Cyclisierung gemäß folgendem Schema:

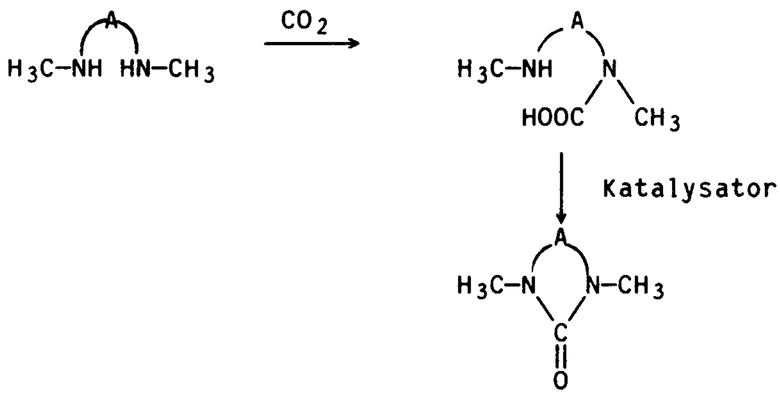


Als Ausgangsstoffe werden Diamine II verwendet, in denen die Reste am Stickstoff Alkylreste mit 1 bis 8 C-Atomen, insbesondere 1 bis 4 C-Atomen, z.B. Methyl-, Ethyl-, Propyl-, Butyl-, Pentyl-, Hexyl-, Heptyl- oder Octylreste bedeuten. Die Ethylenbrücke bzw. Propylenbrücke kann unsubstituiert sein oder 1 bis 6 Alkylreste mit je 1 bis 17 C-Atomen tragen. Vorteilhaft stehen $R^3$ bis $R^8$ für Alkylreste mit 1 bis 12, insbesondere 1 bis 8 C-Atomen in den oben genannten Resten. Wegen ihrer leichteren Zugänglichkeit und im Hinblick auf ihre Verwendung als polare Lösungsmittel werden Ausgangsstoffe mit niedermolekularen Alkylresten $R^1$ bis $R^8$ bevorzugt eingesetzt.

Die Umsetzung der Diamine II mit $CO_2$ kann diskontinuierlich, vorteilhaft kontinuierlich bei vermindertem, leicht erhöhtem oder insbesondere bei Atmosphärendruck durchgeführt werden. Beispielsweise kann man das Diamin kontinuierlich verdampfen, auf die gewünschte Temperatur bringen und mit dem ggf. vorerhitzten $CO_2$-Strom vermischen. Es ist auch möglich zusätzlich ein inertes Trägergas, z.B. Stickstoff oder Argon zu verwenden oder schwerflüchtige Ausgangsstoffe in Gegenwart eines Schleppmittels einzusetzen.

Das Reaktionsgemisch kann im Molverhältnis 1 : 1 zusammengesetzt sein oder einen $CO_2$-überschuß oder einen Diaminüberschuß enthalten. Im allgemeinen wird ein $CO_2$-überschuß von 5 bis 100 Mol%, bezogen auf das Diamin, bevorzugt, um das Diamin vollständig umzusetzen und die freiwerdende Wärme aufzunehmen.

Das Reaktionsgemisch kann vorteilhaft mit einer solchen Geschwindigkeit über den Katalysator geleitet werden, daß etwa 0,5 bis 5 mol Diamin, vorzugsweise 1-3 mol Diamin, pro Liter Katalysator und Stunde umgesetzt werden.

Die Aufarbeitung des anfallenden Umsetzungsgemisches und Isolierung der Produkte kann in an sich bekannter Weise erfolgen, so daß sich nähere Ausführungen hierzu erübrigen. Beispielsweise kann man so verfahren, daß man das Umsetzungsgemisch abkühlt, dabei das gewünschte Produkt zusammen mit dem Reaktionswasser oder nacheinander kondensiert. Das Produkt kann durch Destillation entwässert und gereinigt werden. Gegebenenfalls ist auch nicht umgesetztes oder nur bis zur Carbamidsäure umgesetztes Diamin leicht rückgewinnbar.

Von den erfindungsgemäß herstellbaren Stoffen ist bisher besonders das 1,3-Dimethyl-2-imidazolidinon technisch als Lösungsmittel bzw. Reaktionsmedium genutzt worden. Dieses Produkt hat allerdings den Nachteil, daß es einen Schmelzpunkt von ca. 8 °C besitzt. Bei tieferen Temperaturen läßt es sich daher nicht anwenden oder handhaben. Es wurde nun gefunden, daß das bisher unbekannte 1,3,4-Trimethyl-2-imidazolidinon unerwartet tief, nämlich erst bei -34° C erstarrt und somit die genannten Nachteile vermeidet.

Beispiel 1

Zur Herstellung von 1,3-Dimethyl-2-imidazolidinon wurde als Reaktor ein vertikal stehendes, beheiztes Glasrohr benutzt, dessen oberer Teil (Länge 50 cm) als Verdampfer und Vorheizer und dessen unterer Teil (Länge 100 cm, Volumen 1300 ml) als Festbettreaktor diente. Der obere Teil war mit Glasringen gefüllt und enthielt ein $CO_2$-Einleitrohr, das bis zum Anfang der Katalysatorzone hinabging. Als Katalysator diente $\gamma$-$Al_2O_3$ in Form von Strängen. Am oberen Ende der Glasring-Füllung wurden stündlich 1,5 mol N,N′-Dimethylethylendiamin zugepumpt. Gleichzeitig wurden stündlich 2 mol $CO_2$ durch das Einleitrohr zugeführt. Das Glasrohr wurde auf 250-280° C beheizt. Das entstandene Reaktionsgemisch gelangte vom unteren Ende des Reaktors nach Abkühlung auf Raumtemperatur in einen Flüssigkeitsabscheider. Die gesammelte Flüssigkeit wurde destilliert. Zunächst wurde bei Normaldruck eine Wasserfraktion, dann unter Vakuum das 1,3-Dimethyl-2-imidazolidinon in reiner Form erhalten. Siedepunkt 123-125° C/50 mbar; Schmelzpunkt +8° C. Geringe Mengen des Produktes waren noch aus der Wasserfraktion destillativ zu gewinnen.

Insgesamt betrug die Ausbeute 99 % der Theorie

Beispiel 2

Zur Herstellung von 1,3,4-Trimethyl-2-imidazolidinon wurde die im Beispiel 1 beschriebene Apparatur unter gleichen Bedingungen benutzt, jedoch 1,2-Bismethylaminopropan mit $CO_2$ umgesetzt.
Die Ausbeute an 1,3,4-Trimethyl-2-imidazolidinon betrug 99 % der Theorie.
Die Verbindung siedet bei 225-227° C, der Schmelzpunkt liegt bei -34° C.
Dichte = 1,032 g/ml bei 23° C.

Beispiel 3

Die im Beispiel 1 beschriebene Umsetzung wurde unter gleichen Bedingungen wiederholt, wobei jedoch als Katalysator ein Aluminiumsilikat mit 52 % $SiO_2$ und 48 % $Al_2O_3$ verwendet wurde. Der Umsatzgrad des N,N′-Dimethylethylendiamins betrug ca. 80 %, der Rest wurde zurückgwonnen. Die Selektivität des 1,3-Dimethyl-2-imidazolidinons lag bei 90 %. Ein ähnliches Ergebnis wurde mit einem Kieselsäure-Katalysator erhalten.

Beispiel 4

Analog Beispiel 1 wurde 1,3-Bismethylaminopropan mit $CO_2$ umgesetzt. Die Ausbeute an 1,3-Dimethyltetrahydropyrimidin-2-on betrug 99 % der Theorie.
Die Verbindung siedet bei 229 bis 231° C.

Bei Verwendung eines nur 80 %igen 1,3-Bismethylaminopropans wurde, wenn man diesen Gehalt bei der Berechnung berücksichtigt, ebenfalls eine 99 %ige Ausbeute erzielt.

## Patentansprüche

**1.** Verfahren zur Herstellung von N,N'-dialkylsubstituierten cyclischen Harnstoffderivaten der allgemeinen Formel I

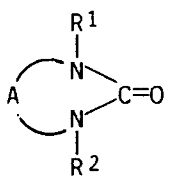

(I),

in der die Reste $R^1$ und $R^2$ Alkylreste mit 1 bis 8 C-Atomen und A eine $CR^3R^4$-$CR^5R^6$- oder eine $CR^3R^4$-$CR^5R^6$-$CR^7R^8$-Gruppe bedeutet, wobei $R^3$ bis $R^8$ für Wasserstoff oder Alkylreste mit 1 bis 17 C-Atomen stehen, durch Umsetzung von Diaminen der allgemeinen Formel II

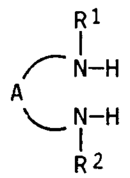

(II)

mit Kohlendioxid, dadurch gekennzeichnet, daß man die Umsetzung in der Gasphase in Gegenwart eines Katalysators von Oxiden von Elementen der dritten oder vierten Hauptgruppe oder zweiten bis sechsten Nebengruppe des Periodischen Systems oder Gemischen hiervon oder in Gegenwart von Aluminium- oder Magnesiumsilikaten durchführt.

**2.** Verfahren nach Anspruch 1, dadurch gekennzeichnet, daß man $\gamma$-$Al_2O_3$ als Katalysator verwendet.

**3.** Verfahren nach Anspruch 1, dadurch gekennzeichnet, daß man Zeolithe als Katalysator verwendet.

**4.** Verfahren nach Anspruch 1 bis 3, dadurch gekennzeichnet, daß $R^1$ und $R^2$ Alkylreste mit 1 bis 4 C-Atomen bedeuten und A eine $CR^3R^4$-$CR^5R^6$- oder eine $CR^3R^4$-$CR^5R^6$-$CR^7R^8$-Gruppe bedeutet, wobei $R^3$ bis $R^8$ für Wasserstoff oder Alkylreste mit 1 bis 8 C-Atomen stehen.

**5.** Verfahren nach den Ansprüchen 1 bis 4, dadurch gekennzeichnet, daß man die Umsetzung bei Temperaturen von 200 bis 350 °C durchführt.

**6.** Verfahren nach den Ansprüchen 1 bis 5, dadurch gekennzeichnet, daß man die Umsetzung kontinuierlich durchführt.

**Claims**

**1.** A process for the preparation of an N,N'-dialkyl-substituted cyclic urea derivative of the formula I

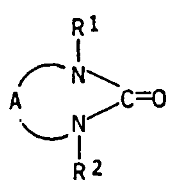

(I)

where $R^1$ and $R^2$ are each alkyl of 1 to 8 carbon atoms and A is $CR^3R^4$-$CR^5R^6$- or $CR^3R^4$-$CR^5R^6$-$CR^7R^8$,
where $R^3$ to $R^8$ are each hydrogen or alkyl of 1 to 17 carbon atoms, by reacting a diamine of the formula II

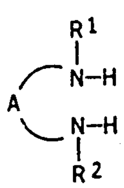

(II)

with carbon dioxide, wherein the reaction is carried out in the gas phase in the presence of a catalyst of an oxide of an element of main group three or four or subgroup two to six of the Periodic Table, or a mixture of these, or in the presence of an aluminum silicate or magnesium silicate.

**2.** A process as claimed in claim 1, wherein the catalyst used is $\gamma$-$Al_2O_3$.

**3.** A process as claimed in claim 1, wherein the catalyst used is a zeolite.

**4.** A process as claimed in any of claims 1 to 3, wherein $R^1$ and $R^2$ are each alkyl of 1 to 4 carbon atoms and A is $CR^3R^4$-$CR^5R^6$ or $CR^3R^4$-$CR^5R^6$-$CR^7R^8$, where $R^3$ to $R^8$ are each hydrogen or alkyl of 1 to 8 carbon atoms.

**5.** A process as claimed in any of claims 1 to 4, wherein the reaction is carried out at from 200 to 350 ° C.

**6.** A process as claimed in any of claims 1 to 5, wherein the reaction is carried out by a continuous procedure.

**Revendications**

**1.** Procédé de préparation de dérivés d'urée cycliques N,N'-dialkylsubstitués de formule générale

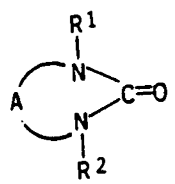

(I),

dans laquelle les restes $R^1$ et $R^2$ représentent des restes alkyle à 1-8 atomes de carbone et A représente un groupement $CR^3R^4$-$CR^5R^6$ ou $CR^3R^4$-$CR^5R^6$-$CR^7R^8$, $R^3$, à $R^8$ étant mis pour des atomes d'hydrogène ou pour des restes alkyle à 1-17 atomes de carbone, par réaction de diamines de formule générale II

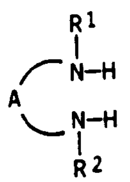

(II)

avec de l'anhydride carbonique, caractérisé en ce qu'on conduit la réaction en phase gazeuse en présence d'un catalyseur d'oxydes d'éléments des groupes IIIA ou IVA ou des groupes IIB à VIB du système périodique ou de mélange de ces oxydes, ou en présence de silicates d'aluminium ou de magnésium.

**2.** Procédé selon la revendication 1, caractérisé en ce qu'on utilise $Al_2O_3$ $\gamma$ comme catalyseur.

**3.** Procédé selon la revendication 1, caractérisé en ce qu'on utilise des zéolithes comme catalyseur.

**4.** Procédé selon l'une quelconque des revendications 1 à 3, caractérisé en ce que $R^1$ et $R^2$ représentent des restes alkyle à 1-4 atomes de carbone et A représente un groupement $CR^3R^4$-$CR^5R^6$ ou $CR^3R^4$-$CR^5R^6$-$CR^{7R8,}$ $R^3$ à $R^8$ étant mis pour des atomes d'hydrogène ou des restes alkyle à 1-8 atomes de carbone.

**5.** Procédé selon l'une quelconque des revendications 1 à 4, caractérisé en ce qu'on mène la réaction à des températures de 200 à 350 ° C.

**6.** Procédé selon l'une quelconque des revendications 1 à 5, caractérisé en ce qu'on mène la réaction en continu.